# EUROPEAN PATENT SPECIFICATION

(11) **EP 0 928 296 B1**
(45) Date of publication and mention of the grant of the patent: **05.12.2001**
(21) Application number: 97910285.2
(22) Date of filing: 11.09.1997
(51) Int. Cl.: C08F 4/00, C07C 255/11, C07C 255/46

(54) **NO-COMPOUNDS FOR PSEUDO-LIVING RADICAL POLYMERIZATION**
NITROSO VERBINDUNGEN ZUR PSEUDO-LEBENDEN RADIKALPOLYMERISATION
COMPOSES DE NO POUR LA POLYMERISATION DE RADICAUX PSEUDO-VIVANTS

(30) Priority: 25.09.1996 EP 96202676
(43) Date of publication of application: 14.07.1999
(73) Proprietor: Akzo Nobel N.V., 6824 BM Arnhem (NL)
(72) Inventor: VERTOMMEN, Luc, Louis, Theophile, NL-6931 VJ Westervoort (NL); VAN DEN HAAK, Hendrik, Jan, Willem, NL-6921 SE Duiven (NL); HOPE, Peter,Nethergate Cottage, Merseyside L37 7A2 (GB); LACROIX, Christine, Pascale, Mireille, NL-7391 WK Twello (NL); MEIJER, John, NL-7415 ES Deventer (NL); TALMA, Auke, Gerardus, NL-7437 AT Bathmen (NL)
(74) Representative: Schalkwijk, Pieter Cornelis, c.s.
(86) International application number: EP9705009
(87) International publication number: WO9813392

(56) References cited:
- EP-A- 0 135 280
- US-A- 5 627 248
- G. MOAD AND E. RIZZARDO: "Alkoxyamine-Initiated Living Radical Polymerisation" MACROMOLECULES, vol. 28, pages 8722-8728, XP002055156 cited in the application
- B.A. GINGRAS AND W.A.WATERS: "Properties and Reactions of Free Alkyl Radicals in Solution" JOURNAL OF THE CHEMICAL SOCIETY, 1954, pages 1920-1924, XP002050740 cited in the application
- A.N. BOYD, F.P. SOUTHERN, AND W.A. WATERS: "Properties and Reactions of Free Alkyl Radicals in Solution - part X" JOURNAL OF THE CHEMICAL SOCIETY, 1958, pages 2056-2058, XP002055157 cited in the application

## Description

The present invention relates to the use of NO-compounds as initers in pseudo-living radical polymerization processes, as well as to the polymers formed in such processes and to certain novel NO-compounds.

Currently, numerous institutes are investigating so-called "pseudo-living polymerization" processes. Such polymerization processes allow the production of polymers with a very narrow molecularweight distribution as well as of specific Mock-copolymers. Living polymerization processes have long been known from ionic processes such as the anionic polymerization of styrene. There is great interest in (pseudo) living polymerizations using free radicals, because such polymerizations would not suffer from many of the problems associated with living ionic polymerizations, for instance, the need to use reactants of very high purity to avoid poisoning the reactive centre.

The current state of the art for (pseudo-) living polymerization processes involving radicals typically requires the use of a "stable free radical agent" in combination with an initiator, as described, for instance, in the recently issued patents US 5,498,679, US 5,449,724, US 5,412,047, and US 5,312,871, as well as in patent applications WO 95/26987 and WO 94/11412. The stable free radicals of choice are usually compounds possessing stable nitroxide radicals. However, the synthesis of such stable free radical agents, as described, for instance, in *Macromolecules* **1996**, 29, 3323-3325, is cumbersome, and the nitroxide radicals thus obtained are quite expensive. The initiator in such prior art processes is typically used in such a quantity that the ratio of free radical agent to the amount of radicals generated by the initiator is not unity, see for example WO 94/11412, and many side reactions take place, leading to both undesired side products and a reduced initiator efficiency. The reduced initiator efficiency is often counterbalanced by using an excess of this product, with all associated disadvantages.

As an improvement on these known pseudo living radical polymerization processes, US 4,581,429 describes how nitroxide stable free radical compounds can be reacted with azo-initiators or with unsaturated compounds in combination with an initiator. The resulting alkoxyamines can be used in what are called "controlled-growth free radical polymerization" reactions of a "living" nature. This concept was further elaborated on in *Macromolecrules*, **1995**, 28, 8722-8728, and *Macromolecules* **1996**, 29, 5246-5254, where it is demonstrated "how the alkoxyamine structure determines the C-O bond strength and what is required for these species to be successful initiators of living radical polymerization." In the former article it is disclosed that electron donating or withdrawing groups - i.e., electron donating or withdrawing as compared to alkyl groups - on the nitroxide are undesired since they are "unlikely to be useful in living polymerization." Evaluation of the disclosed alkoxyamine compounds showed that they do not perform to full satisfaction in the polymerization. More particularly, both the polymer yield and control of the molecular weight of this polymer needed improvement. Also, not all desired monomers could be polymerized in a controlled way.

The present invention relates to processes and NO-compounds which serve to avoid or minimize the disadvantages as reported for current polymerization processes and existing NO-compounds.

Accordingly, it is the primary object of this invention to provide a pseudo-living polymerization process in which specific NO-compounds are combined with one or more of various monomers with subsequent polymerization at a variety of temperatures.

It is another object of the invention to provide an economical polymerization process in which no additional initiators are used and no chain transfer agent or other means is required to produce polymers with a controlled molecular weight in high yields.

A third object of the invention is to provide an economical process to produce block copolymers using NO-compounds in pseudo-living polymerization processes. These copolymers can be, for instance, block, star, and/or comb copolymers.

A further object of the invention is to provide certain NO-compounds that can be used as initers in pseudo-living polymerization processes in which a variety of monomers can be polymerized in high yields, with accurate control of the molecular weight of the formed polymer, at various temperatures, with NO-compounds that are easy to synthesize.

Still another object of the invention is to provide a polymer that can easily be recycled to its monomeric compound(s) because of the NO end group of the polymer. This will make it possible for the polymers to be recycled at fairly low temperatures without the addition of high-temperature initiators, thus allowing a more economical and environmentally-friendlyrecycling process.

These and other aspects of the invention are outlined in more detail below.

### Description of the invention

As used herein, the term "pseudo-living polymerization process" stands for the radical polymerization process in which one or more ethylenically unsaturated monomers are polymerized by means of at least one initer. According to a non-limiting theory, the polymerization kinetics in these novel processes depend, amongst others, on the thermal decomposition rate of the initer that is used. It is noted that the polymerization will not be of a perfect "living" nature. Also, it was observed that these polymerization kinetics are not necessarily the same as in conventional "pseudo-living" processes.

The term "initer" as used herein defines NO-compounds of a specific structure, as defined below. Such NO-compounds are referred to here as initers, for it is believed that the carbon-centred radical formed upon decomposition initiates the polymerization, whereas the nitroxide radical will terminate the growing chain. Without the invention being limited to such a theory, it is further believed that the termination reaction is reversible at the polymerization temperature, so that monomers can be inserted between the last monomeric unit and the nitroxide moiety of the initer.

The first embodiment of the invention relates to a polymerization process in which at least one NO-compound with at least one moiety according to formula (I) or (II), 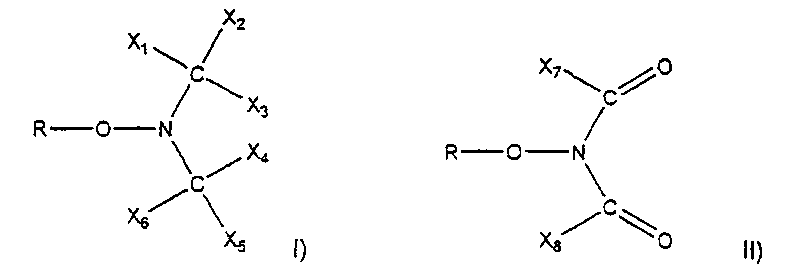 wherein:
- R represents a group which has at least one carbon atom and is such that the free radical R• is capable of initiating the free radical polymerization of unsaturated monomers;
- at most five of the groups represented by X₁-X₆ are the same or different straight-chain or branched, substituted or unsubstituted (cyclo) alkyl groups, while two or more of the groups X₁-X₆ may be linked to form cyclic structures,
- the complementary groups X₁ through X₆ are functional groups selected from substituted or unsubstituted phenyl, cyano, ether, hydroxy, nitro, dialkoxyphosphonyl, and carbonyl containing groups, or, alternatively, -CX₁X₂X₃ and/or -CX₄X₅X₆ represent a phenyl group,
- X₇ and X₈ are independently selected from alkyl, aryl, alkaryl, and aralkyl, while X₇ is optionally linked with X₈ to form bridged structures, and is combined with an ethylenically unsaturated monomer composition to be polymerized.

Preferred examples of carbonyl containing functional groups are ester, carboxyalkyl, aldehyde, anhydride, and ketoalkyl groups.

Although X₁-X₆ may be linked to form cyclic structures, they preferably are not so linked. Also, it is preferred that each of X₁-X₈ contains fewer than 30 carbon atoms. More preferably, each of X₁-X₈ contains fewer than 10 carbon atoms in order to produce initers with a low molecular weight. Most preferably, at most five of X₁-X₆ are independently selected from methyl, ethyl, propyl, isopropyl, butyl, sec.butyl, tert.butyl, and cyclohexyl. X₇ and X₈ are preferably linked. More preferably, X₇ and X₈ are part of one substituted or unsubstituted phenyl or naftyl group. Most preferably, X₇ and X₈ ar part of one phenyl group.

Furthermore, it is preferred that the process involves initers with a moiety of formulae I or II, with the functional group being selected from phenyl, cyano, dialkoxyphosphonyl, carboxyalkyl, and ester. More preferred is a process where the initer possesses a cyano, phenyl, ester or dialkoxyphosphonyl group. Even more preferred, is a process where the initer possesses a cyano or phenyl group. Most preferred is a process with at least one inter with a cyano moiety. Preferably, these initers can be produced without toxic byproducts being formed. In this respect, it is noted that initers derived from 2,2'-azobis(isobutyronitril) are less favoured because the toxin tetramethylsuccinonitril is a known decomposition product of this material.

Non-limiting examples of functional groups of the initer further include methylether, ethylether, propylether, butylether, poly(alkylether), methylketone, ethylketone, propylketone, isopropylketone, butylketone, isobutylketone, tent.butylketone, diethoxyphosphonyl, ethoxypropoxyphosphonyl, dipropoxyphosphonyl, dibutoxyphosphonyl, diisobutoxyphosphonyl,-C(O)OCH₃, -C(O)OC₂H₅, and C₁-C₂₀ carboxylic acid esters.

The R group may bear one or more of the ONC(X₁₋₃)C(X₄₋₆) functions as long as each of the radicals R• formed upon scission of one or more of the R-O bonds is capable of initiating the free radical polymerization of an unsaturated monomer. Furthermore, in the first instance it is preferred that R is not of a polymeric nature, meaning that R preferably does not comprise more than approximately 4 recurring units incorporated by radically polymerizing one or more unsaturated monomers. Preferably, R does not contain more than two of such recurring units. Most preferred are compounds wherein R equals C(X₁₋₃) or C(X₄₋₆). However, the polymer that results from the polymerization process can be reused as an initer according to the invention. Therefore, R is not limited to a non-polymeric group.

The NO-compounds can be used at various temperatures, with the choice of NO-compound depending on the desired polymerization temperature. In this respect it is noted that radical polymerization reactions of (meth)acrylic monomers are typically conducted at lower temperatures than (co)polymerizations involving a styrene monomer. For other, regularly used, monomers, suitable polymerization temperatures are also well-known. The NO-compound of choice has an acceptable decomposition rate at the desired polymerization temperature, which is generally between 50 and 180°C. From, for instance, *Macromolecules*, **1995**, 28, 8722-8728, it is known that the use of electron withdrawing functional groups will stabilize the R-O bond, whereas electron donating functional groups will labilize it. Given this information and the teaching of this document, the man skilled in the art will have no problem selecting suitable types and numbers of functional groups to obtain usable NO-compounds. Before conducting actual polymerization experiments, the half-life of the NO-compound may be determined in one or more well-known ways.

The invention is not limited to the use of NO-compounds alone. Also encompassed is the combination of such an NO-compound with additives catalyzing its decomposition.

Preferably, the process according to the invention results in polymers with a number average molecular weight (Mn) close to the theoretical value (Mth), calculated from [monomer]/[initer]∗Mₘₒₙₒₘₑᵣ. More particularly, it is preferred that the Mn does not exceed the Mth by 15 times. Most preferred is a process wherein Mn is less than five times Mth.

The ethylenicalty unsaturated monomers that can be polymerized and/or copolymerized by means of the NO-compounds include: styrene, divinylbenzene, α-methylstyrene, chloromethylstyrene, acrylic acid, esters of acrylic acid, methacrylic acid, esters of methacrylic acid, maleic acid, maleic anhydride, esters of maleic acid, fumaric acid, esters of fumaric acid, acrylonitrile, vinylpyridine, isoprene, butadiene, trienes, styrene phosphonic acid, vinyl phosphonic acid, styrene phosphonic esters, vinyl phosphonic acid esters, maleimides, citraconimides, itaconimides, and vinylacetate. Also derivatives of these monomers obtained, for instance, by substituting a functional group for a proton, can be used. Examples of suitable functional groups are halogen, hydroxy, amino, methoxy, nitro, carboxy, and cyano, but the invention is not limited to these.

In a second embodiment, the invention relates to a process in which an inter according to formula (I) or (II) is combined with a monomer composition to produce (co)polymers with a controlled molecular weight in good yield, without additional radical generating species or chain transfer agents, which are known from classic radical polymerization processes, being introduced in the process. Examples of radical generating species include, organic peroxides, azo-initiators and UV-initiators. The use of one or more initers with at least one moiety according to formula (I) or (II) in polymerization processes obviates such radical generating species or chain transfer agents because they allow a very high polymerization rate while the degree of polymerization (the number of polymerized monomer units in the final polymer) remains close to the calculated value of the molar ratio of monomer to initer. The dispersity of the molecular weight distribution of the polymer will typically not be close to unity as in living polymerizations, but may vary from, for instance, 1.4 to 2.7. This range is indicative only; especially when block copolymers are made by sequentially adding monomers, higher dispersity values have been observed.

Should the molecular weight of the polymer and/or its dispersity become too high, for instance, when excessive thermal polymerization of one or more of the monomers takes place, then the initer may be combined with a stable free radical agent. In such a case, the stable free radical agent preferably will have the same structure as the nitroxide radical that results from thermal decomposition of the NO compound according to the invention.

In a third embodiment, the invention relates to polymers which are obtainable by the polymerization processes according to the invention and will have one alkoxyamine end group, said end group being of the structure -ON(CX₁X₂X₃)CX₄X₅X₆ or -ON(C(O)X₇)C(O)X₈, wherein X₁-X₈ are defined as above. Consequently, these polymers are themselves initers according to the invention. Hence, they can be used in further pseudo-living polymerization reactions in order to make, for instance, block copolymers. Before being used in such a fashion, the polymers may first be collected, purified, and dried. Preferably, however, the fresh monomer or monomer mixture can be added directly to the reaction mixture when it contains the desired polymer, for example, when a desired conversion, for example 90 percent by weight (%w/w), of the first monomer or monomer mixture is attained. Depending upon the reaction kinetics of the monomers involved, the presence of two or more monomers at the same time may result in a monomer distribution within the growing polymer segment of a random or more regular (for instance, alternating) nature. In this way, it is possible to produce (block) copolymers of varying nature in an economical fashion. Since the (block) copolymer obtained in turn is an initer according to the invention, subsequent polymerization steps are possible. Preferably, however, the initers according to the invention are low-molecular weight materials. More preferably, such initers have fewer than four recurring monomer units, since such products are easier to handle. With the initers and the process according to the invention one can produce block copolymers with specifically designed block compositions and sequences, resulting in materials with specific properties. They may be used as, for example, compatibilizers, coupling agents, dispersing agents for filled thermoplastics and SiO₂ filled rubber, telechelic slip agents, impact modifiers, anti-static agents, sizing agents, emulsifiers/surfactants, pigment dispersants, lubricants, processing aids, thickeners, adhesives, oil additives, and/or additives for the preservation and hardening of wood.

In a further embodiment, the invention relates to specific NO-compounds containing at least one moiety of the formula (I) which are pre-eminently suited to be used in the polymerization process according to the invention, wherein:
- R represents a group which has at least one carbon atom and is such that the free radical R• is capable of initiating the free radical polymerization of unsaturated monomers;
- at most five of the groups represented by X₁ through X₆ are the same or different straight-chain or branched substituted or unsubstituted (cyclo) alkyl groups, while two or more of the groups may be linked to form cyclic structures,
- the complementary groups X₁ through X₆ are functional groups selected from substituted or unsubstituted phenyl, cyano, ether, dialkoxyphosphonyl and carbonyl containing groups, such as ester, anhydride, and keto groups, and
- R is not polymeric when -N(CX₁X₂X₃) CX₄X₅X₆ is 2,5-dimethyl-2,5-diphenylpyrrolidin-1-,
with the proviso that the NO-compound is not tris-(2-cyano-2-propyl)-hydroxylamine, tris-(2-carboxyethyl-2-propyl) hydroxylamine, and/or tris-(2-carboxymethyl-2-propyl)hydroxylamine.

Incidentally, it is noted that certain specific alkoxyamines are known which are not mentioned in US 4,581,429. Gingras and Waters describe the preparation of tris-(2-cyano-2-propyl) hydroxylamine in *J. Chem. Soc.,* **1954,** 1920-1924. Tris-(2-carboxymethyl-2-propyl)hydroxylamine is synthesized as described by Boyd in *J. Chem.* Soc., **1958,** 2056, and tris-(2-carboxyethyl-2-propyl) hydroxylamine is synthesized by Masui in *Tetrahedron*, **1965**, 21, 2831. Also, the polymers mentioned in *Macromolecules* **1996**, 29, 3323-3325 and *J.M.S.-Rev.Macromol.Chem.Phys*, **1994,** 34(2), 288-289 are accidental anticipations of some initers according to the invention.

Upon thermal decomposition, with the R-O bond being broken, the NO-compounds according to formula (I) will generate two radicals. The carbon centred R• radical can initiate a polymerization reaction. However, during such a polymerization the nitroxide radical will terminate the growing chain, forming a new initer which in tum is thermally labile and may be involved in subsequent polymerization reactions. In this way a pseudo-living radical polymerization will take place in which monomeric units are inserted at the nitroxide terminated end of the polymer. Because of the nature of such a process, the polymer that is formed will have a polydispersity (D) typically greater than unity (1.0), the dispersity observed in regular living polymerization reactions.

The compounds according to the invention can be produced in various conventional ways. It was surprisingly found that the reaction of carbon-centred radicals and nitric oxide (NO), which has been known since 1954 (see, for instance, *J. Chem. Soc*., **1954**, 1920-1924), results in a very economical route to effective initers. Reactants and reaction conditions are chosen so that during the synthesis at least one of the two carbon-centred radicals attached to the nitrogen atom will bear a functional group. This is most easily achieved by selecting the proper concentration of one or more of the functional group-bearing carbon-centred radical precursors. Preferably, the functional group-bearing carbon-free radical is obtained by the decomposition of one or more appropriate azo, C-C or other initiators such as diacyl peroxides with a high decarboxylation rate. Depending on the use of the initer, it may be preferable to react only one type of carbon-centred radical (containing a functional group) with the NO gas.

Alternatively, compounds according to the invention can be produced by the well-known reaction of a nitroso compound under influence of heat or with carbon-centred radicals. Depending on the structure of the nitroso compound, the man skilled in the art will have no problem selecting a suitable carbon-centred radical generating species in order to arrive at compounds according to the invention. An example of such a process is the preparation of N-tert.-butoxy phthalimide (BuPI), according to formula (II), and of the structure 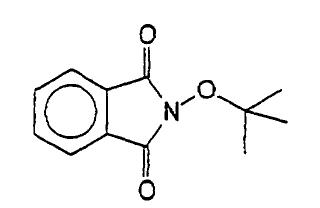 as described by T. Kolasa, A. Chimiak and A. Kitowski in *J. Prakt. Chem.,* **1975**, 317, 252-256.

The (block) (co)polymers that are made by the process of the present invention can fairly easily be recycled into low-molecularweight fragments. In contrast to current recycling processes as, for instance, known from European patent application EP-A-0273274 and non-prepublished European patent application 96202111.9, filed on July 25, 1996, large amounts of compounds capable of generating free radicals and very high temperatures are not needed. The low-molecularweight fragments that are formed can be used as a fuel. Hence, the (block) (co)polymers according to the invention can be recycled using less energy and without the use of specific additives. Preferably, the temperature in this recycling process is kept below 300°C.

The invention will be further illustrated by the following examples.

### Experimental

The chemicals used in the following examples were all reagent grade unless specified otherwise. Styrene and 1,2-dichlorobenzene (ODCB) were supplied by Baker (Baker PA grade). Styrene was freed of inhibitor by a treatment with an alkaline Al₂O₃ column. Methyl methacrylate (MMA), 2,2,6,6-tetramethyl-1-pyrrolidinyloxy (TEMPO®), and (+)camphorsulfonic acid (CSA), an agent which is presumed to reduce thermal polymerization in pseudo-living radical polymerizations, were supplied by Aldrich., Perkadox® AIBN (2,2'-azobis(isobutyronitrile]), Perkadox® AMBN (2,2'-azobis[2-methylbutyronitrile]), and all other initiators were supplied by Akzo Nobel or synthesized as laid down in the available documentation. NO gas was supplied by Indugas. Tinuvin® 123 is a product of Ciba Geigy.

Cumyl-TEMPO as used in the comparative examples was synthesized following the general procedure of EP 0 309 402, Example 63. More specifically, 0.454 mol of tent.butylhydroperoxide was dosed in five hours to a refluxing solution (atmospheric pressure) of 0.14 mol tetramethylpiperidine, 1.582 mol cumene, and 4 mmol MoO₃, and the mixture was reacted further at this temperature for 12 hours. The reaction product was reacted with 0.17 mol of di-tert.butylperoxide in an autoclave at 140°C for four hours. Cumene was removed using a rotary evaporator, and cumyl-TEMPO was obtained by crystallization.

For polymerizations conducted in sealed tubes, the reaction mixture was degassed by means of well-known freeze-thaw techniques, after which the ampoule was sealed under vacuum. For polymerizations conducted in an open flask, a three-necked flask was fitted with a (gas) inlet, a stirrer, and a condenser. The contents of the flask were flushed with Argon before and during the polymerization.

The conversion of monomer into polymer was determined in conventional ways by gas chromatographic (GC) analysis for monomer, using monochlorobenzene as the internal standard. Samples were taken by either breaking an ampoule or through one of the necks of the flask, using a syringe. The reactants were quenched by rapid cooling and dissolved in dichloromethane (for GC analysis) or THF (for molecular weight analysis).

The number average molecular weight (Mn), weight average molecular weight (Mw), and dispersity (D) of the polymers were analyzed in conventional ways by means of size exclusion chromatography, using the THF solution as the eluent. Polystyrene samples were used as calibration standards.
Polymers were isolated from the solution by precipitation in n-pentane and subsequent drying in a vacuum oven at 50°C to constant weight (20-100 hours).

Polymers were characterized by means of 300 or 400MHz ¹H and 100 MHz ¹³C NMR.

No attempt was made to optimize yields.

### Example 1

Preparation of NO-compounds using NO gas
a) N,N,O-Tris-(1-cyano-1-methylethyl)hydroxylamine((IBN)₃NO) 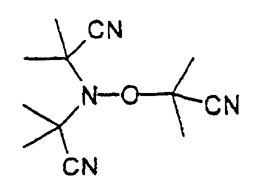
   A solution of Perkadox AIBN (152.7 gr, 0.93 mol) in 1500 ml of toluene was stirred, flushed with nitrogen gas, and heated to 76°C. A stream of nitrogen monoxide (5 l/hr) was passed through the solution, and a deep greenish/blue colour appeared. Heating was continued for 2 hours at 76°C, 1 hour at 82°C, 30 minutes at 88°C, 15 minutes at 94°C, and finally 15 minutes at 119°C to achieve complete decomposition of the AIBN. Residual nitrogen monoxide was removed by flushing the reaction mixture with nitrogen gas, at which point the colouration changed to yellow. The solvent was removed in a rotary evaporator, and the resulting solution was steam-distilled to remove the by-product tetramethyl succinonitrile. Next, the water/product mixture was left to cool down to room temperature, and the product precipitated. This solid was removed by filtration, dissolved in dichioromethane, and then dried with magnesium sulfate. Evaporation of the solvent gave 84.3 g (58%) of a yellow solid, the (IBN)₃NO.
b) N,N,O-Tris-(1-cyano-1-methylpropyl)-hydroxylamine((MBN)₃NO) 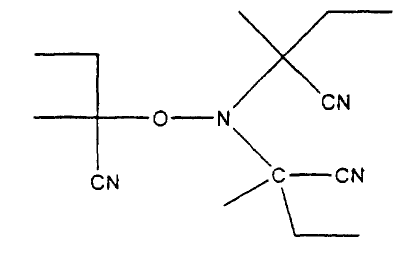
   A solution of Perkadox AMBN (178.8 gr, 0.93 mol) in 1500 ml of toluene was stirred, flushed with nitrogen gas, and heated to 76°C. A stream of nitrogen monoxide was passed through the solution, and a deep greenish/blue colour appeared. Heating was continued for 2 hours at 76°C, 1 hour at 82°C, 30 minutes at 88°C, 15 minutes at 94°C, and finally 15 minutes at 119°C to achieve complete decomposition of the AMBN. Residual nitrogen monoxide was removed by flushing the reaction mixture with nitrogen gas, at which point the colouration changed to yellow. The solvent was removed using a rotary evaporator, and the resulting solution was steam-distilled in order to remove the by-product hexane-3,4-dinitrile. Next, the water/product mixture was left to cool down to room temperature. The solution was extracted with ether, and the organic layer was dried with magnesium sulfate. Evaporation of the solvent gave 85.8 g (50%) of a yellow liquid, the (MBN)₃NO.
c) N,N.O-Tris-(1-cyano-1-cyclohexyl)hydroxylamine ((CCH)₃NO) 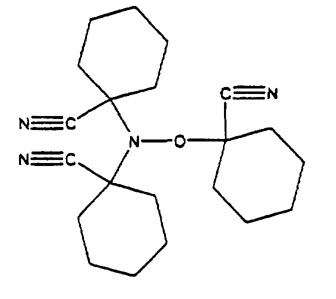
   A solution of 30 g (0.123 mol) 1,1'-azobis(1-cyanocyclohexane)in 130 ml of toluene was stirred, flushed with nitrogen gas, and heated to 76°C. A stream of nitrogen monoxide (5 l/hr) was passed through the solution until a constant blue colour appeared. Heating was continued for 2 hours at 100°C, 1 hour at 107°C, 30 minutes at 112°C, 15 minutes at 118°C, and finally 15 minutes at 125°C. Residual nitrogen monoxide was removed by flushing the reaction mixture with nitrogen gas. The solvent was removed in a rotary evaporator, and the resulting solution was steam-distilled to remove the by-product di(cyclohexylnitrile). Next, the water/product mixture was left to cool down to room temperature, and the product precipitated. This solid was removed by filtration, dissolved in dichloromethane, and then dried with magnesium sulfate. Evaporation of the solvent gave 15.3 g (53%) of an off-white powder, the (CCH)₃NO.

### Example 2

### Preparation of NO-compounds involving nitroso compounds

### Bis-(2-cyano-2-propyl)-N-phenylhydroxylamine ((IBN)₂ArNO)

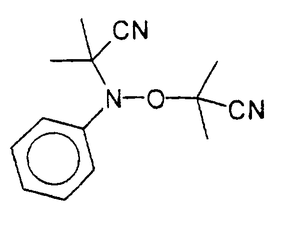

A suspension of Perkadox AIBN (65.6 gr, 0.4 mol) in 100 ml of toluene was added to a refluxing solution, at atmospheric pressure, of nitrosobenzene (21,4 g, 0.2 mol) in toluene (400 ml), with the aid of a Watson Marlow pump. The solution turned greenish/blue. AIBN was dosed during a period of 80 minutes. Heating was continued for 1 hour to complete decomposition of the AIBN. The solution became yellow. After steam-distillation, the residue was twice recrystallized in ether. 16 g (35%) of a yellow powder were obtained.

### Example 3

Styrene, in a concentration of 4.1 mol/l in ODCB, was polymerized in a stirred open flask under an argon atmosphere, at a temperature of 140°C for 6 hours. (IBN)₃NO was used in a concentration of 0.087 mol/l, and CSA was present in a concentration of 0.027 mol/l. Samples were taken every 30 minutes. Analyses of the samples gave the following results:

| Time | Conversion | Mn |
|---|---|---|
| (min) | (% w/w) | (dalton) |
| 0 | 0 | |
| 30 | 30.1 | 1430 |
| 60 | 45.9 | 2120 |
| 90 | 56.9 | 2550 |
| 120 | 65.7 | 2950 |
| 150 | 71.8 | 3100 |
| 180 | 76.7 | 3310 |
| 210 | 80.3 | 3400 |
| 240 | 83.1 | 3520 |
| 270 | 85.4 | 3530 |
| 300 | 86.7 | 3680 |
| 330 | 88.1 | 3670 |
| 360 | 89.3 | 3670 |

Extrapolation of the Mn to 100 % conversion results in values close to the theoretical value for pseudo-living polymerizations, viz. 4200 vs. [M]/[I]∗M_{styrene} =4900. The deviation is believed to have been caused by some polymerization of styrene monomer at the very beginning of the polymerization process. The polymer was retrieved and characterization showed it to contain the expected bis(2-cyano-2-propyl)-hydroxylamine groups.

### Comparative Example A

Styrene (bulk = 8.7 mol/l) was polymerized in a stirred open flask at 130°C under argon, using dibenzoyl peroxide (BPO) in a concentration of 0.072 mol/l and 2,2,6,6-tetramethylpiperidinoxy free radical (TEMPO), with the following results.

| Time | Conversion | Mn | D |
|---|---|---|---|
| (min) | (%w/w) | (Dalton) | |
| 0 | 0 | | |
| 60 | 39 | 6090 | 1.33 |
| 120 | 44 | 7350 | 1.35 |
| 180 | 47 | 8430 | 1.37 |
| 240 | 65 | 12180 | 1.43 |
| 300 | 68 | 12810 | 1.43 |
| 360 | 82 | 15840 | 1.42 |

In this polymerization employing both a stable free radical agent and an initiator, pseudo-living polymerization kinetics were also observed. Also this polymer was analyzed after precipitation. The Tempo endgroups were clearly detectable as well as the benzoyl end groups.

### Examples 4-8 and Comparative Examples B-E

Pyrex tubes containing styrene, an NO-compound, and optionally CSA and/or ODCB were degassed using a freeze-thaw method and sealed under vacuum. The reaction mixture was polymerized at 140°C. The concentration of the NO-compound was selected so as to give the indicated theoretical number average molecular weight (Mth) at 100% monomer conversion. In Examples 4 and 7 the styrene was mixed with ODCB in a 50/50 ratio (by weight). The conversion and the molecular weight (distribution) of the polymer were determined after 6 hours of reaction time, with the following results:

| Exp. | Initer | CSA | Mth | Mn | Conversion | D |
|---|---|---|---|---|---|---|
| | | (mol/l) | (Dalton) | (Dalton) | (%w/w) | |
| 4 | BuPI | 0.027 | 4000 | 50300 | 30.1 | n.d. |
| 5 | (IBN)₂ArNO | 0.027 | 5000 | 30000 | 97.5 | 2.2 |
| 6 | (IBN)₃NO | 0.027 | 5000 | 4800 | 98.2 | 3.3 |
| 7 | (MBN)₃NO | 0.027 | 4000 | 3600 | 100.0 | 1.7 |
| 8 | (CCH)₃NO | 0.053 | 4300 | 3875 | 98.6 | 2.3 |
| B | None | 0 | - | 143400 | 90.9 | 2.4 |
| C | None | 0.027 | - | 173000 | 37.1 | 3.1 |
| D | Cumyl-TEMPO | 0.053 | 5000 | 119000 | 71.7 | 2.6 |
| E | Tinuvin 123 | 0.053 | 1000 | 17000 | n.d. | n.d. |
| n.d. = not determined | | | | | | |

Clearly, the initer of choice has a pronounced effect on the molecular weight of the polymer.

### Examples 9-11 and Comparative Examples F-H

Example 4 was repeated using methyl methacrylate (MMA) (bulk = 9.3 mol/l) instead of styrene. Initers were selected and used in such a concentration as to give a polymer with Mth=5000, except in Example 11, where Mth=4000. CSA was used in a concentration of 0.027 mol/l in Examples G and 9-11. CSA was absent from Example F. In Example 11, the MMA was mixed with ODCB in a ratio of 50/50 (by weight). The following was observed after 6 hours of polymerization time.

| Experiment | Initer | Mn | Conversion | D |
|---|---|---|---|---|
| | | (Dalton) | (%w/w) | |
| 9 | (IBN)₂ArNO | 13670 | 76.6 | 1.9 |
| 10 | (IBN)₃NO | 1892 | 79.4 | 2.6 |
| 11 | (MBN)₃NO | 3400 | 93.0 | 1.7 |
| F | None | 1106000 | 8.8 | 2.4 |
| G | None | 951250 | 6.6 | 2.1 |
| H | Cumyl-TEMPO | 205000 | 78.1 | 2.4 |

Again, the influence of the initers according to the invention on both the conversion rate and the molecular weight of the polymer is pronounced. Also, it was observed that the use of other TEMPO based initers did not allow control of the molecular weight.

### Example 12

In sealed tubes, a solution of 4.4 mol/l of MMA in ODCB was polymerized at 140°C for 6 hours. (IBN)₃NO was used as initer at a concentration of 0.1 mol/l. CSA was present in a concentration of 0.027 mol/l. The resulting polymer was isolated and characterized with ¹H-NMR.

| Time | Conversion | Mn | D |
|---|---|---|---|
| (min) | (%w/w) | (Dalton) | |
| 0 | 1.5 | - | |
| 45 | 84.3 | 2560 | 1.7 |
| 90 | 84.5 | 2545 | 1.7 |
| 135 | 84.4 | 2520 | 1.7 |
| 180 | 84.1 | 2550 | 1.7 |
| 225 | 84.6 | 2580 | 1.7 |
| 270 | 84.6 | 2565 | 1.7 |
| 315 | 84.3 | 2640 | 1.7 |
| 360 | 84.3 | 2615 | 1.7 |

The polymerization rates were exceptionally high. As in Examples 10 and 11, a molecular weight lower than the theoretical value was observed. One of the reasons for this may have been the fact that polystyrene standards were used for calibration of the size exclusion chromatograph. NMR showed the expected structure.

### Example 13

In an open flask, a solution of 4.7 mol/l of MMA in ODCB was polymerized at 120°C for 6 hours. (IBN)₃NO in a concentration of 0.12 mol/l and CSA in a concentration of 0.028mol/l were used. The final polymer was isolated and analyzed. The following results were obtained:

| Time | Conversion | Mn | D |
|---|---|---|---|
| (min) | (%w/w) | (Dalton) | |
| 0 | 1.0 | - | |
| 15 | 55.7 | 3300 | 1.4 |
| 30 | 75.0 | 3335 | 1.4 |
| 45 | 81.1 | 3330 | 1.4 |
| 60 | 84.3 | 3260 | 1.4 |
| 75 | 86.2 | 3250 | 1.5 |
| 90 | 87.4 | 3190 | 1.5 |
| 105 | 88.0 | 3200 | 1.5 |
| 120 | 88.4 | 3240 | 1.5 |
| 240 | 89.9 | 3040 | 1.5 |
| 300 | 91.3 | 3030 | 1.5 |
| 360 | 91.3 | 3050 | 1.5 |

The results are comparable with the results from the previous example.

### Examoles 14-18 and Comparative Examples I and J

Example 4 was repeated using butyl acrylate (BA) instead of styrene and a polymerization temperature of 120°C. CSA was used in a concentration of 0.05 mol/l in all examples, except for Examples 14 and 16. The following was observed after 6 hours of polymerization time.

| Experiment | Initer | Mth | Mn | Conversion | D |
|---|---|---|---|---|---|
| | | (Dalton) | (Dalton) | (%w/w) | |
| 14 | (IBN)₃NO | 6432 | 6470 | 99.7 | 2.4 |
| 15 | (IBN)₃NO | 6184 | 6100 | 99.7 | 2.4 |
| 16 | (MBN)₃NO | 6066 | 5474 | 99.7 | 8.1 |
| 17 | (MBN)₃NO | 5926 | 5426 | 99.7 | 8.6 |
| 18 | (IBN)₂ArNO | 6531 | 53000 | 43.5 | 4.7 |
| I | None | - | 200000 | 44.6 | 2.8 |
| J | Cumyl-TEMPO | 5766 | 68900 | 16.6 | 2.2 |

These experiments show that butyl acrylate can also be polymerized efficiently.

### Examples 19 and 20

In Examples 19 and 20, isoprene in ODCB (4.87 mol/l) was polymerized using 0.123 mol/l of (IBN)₃NO and (MBN)₃NO, respectively (Mth=2700) in accordance with the procedure of Example 4, except that the polymerization temperature was 130°C. At some intervals, the content of a tube was analyzed, with the following results.

### Example 19

| Time | Conversion | Mn | D |
|---|---|---|---|
| (min) | (%w/w) | (Dalton) | |
| 60 | 26.3 | 4850 | 1.5 |
| 120 | 30.1 | 4850 | 1.6 |
| 180 | 36.3 | 4880 | 1.7 |
| 240 | 46.4 | 4930 | 1.7 |
| 300 | 52.1 | 4800 | 1.7 |
| 360 | 55.8 | 4860 | 1.7 |

### Example 20

| Time | Conversion | Mn | D |
|---|---|---|---|
| (min) | (%w/w) | (Dalton) | |
| 60 | 27.5 | 31200 | 1.5 |
| 120 | 38.1 | 3180 | 1.6 |
| 180 | 49.4 | 3330 | 1.7 |
| 240 | 59.2 | 3320 | 1.7 |
| 300 | 64.2 | 3470 | 1.7 |
| 360 | 68.6 | 3460 | 1.7 |

These experiments show that isoprene can also be polymerized efficiently.

### Examples 21-28

Using pyrex tubes, styrene (4.12 mol/l) in ODCB was polymerized for six hours using 0.12 mol/l of (IBN)₃NO, (Mth = 3550) as described in Example 4, at a temperature as specified in the following table. In Examples 21-24 no CSA was used, while in Examples 25-28 the concentration of CSA was 0.027 mol/l.

| Example | Temperature | Conversion | Mn |
|---|---|---|---|
| | (°C) | (%) | (Dalton) |
| 21 | 80 | <1 | 950 |
| 22 | 100 | 4.5 | 1050 |
| 23 | 120 | 41.2 | 3000 |
| 24 | 150 | 99.0 | 3390 |
| 25 | 80 | <1 | 1000 |
| 26 | 100 | 4.6 | 970 |
| 27 | 120 | 36.3 | 2780 |
| 28 | 150 | 98.0 | 2280 |

Repeating these experiments with (MBN)₃NO gave comparable results, except that faster polymerization rates were observed.

### Examples 29-31 and Comparative Example K

In these examples the performance of initers in a polymerization to make high solid acrylic resins was evaluated. To this end, using a stirred glass reactor equipped with a condenser, a mixture consisting of 40 g butyl acrylate, 28 g hydroxyethyl methacrylate, 20 g styrene, 10 g methyl methacrylate, 2 g maleic anhydride, and 21.4 meq. initer or 30.0 meq. tert.butylperoxy 2-ethylhexanoate (Trigonox® 21) (see table) was metered to 40 g solvent (see table), in a nitrogen atmosphere at atmospheric pressure, in four hours. During the addition the temperature of the reaction mixture was maintained at 126 or 140°C (see table). After the addition, the reaction mixture was further reacted for one hour at the specified temperature. The following results were obtained after analysis of the product.

| Example | Radical | reaction | solvent | solids | Mn | Disp |
|---|---|---|---|---|---|---|
| | source | temp. (°C) | | (%) | (Dalton) | |
| 29 | (IBN)₃NO | 126 | nBuAc | 66.0 | 4100 | 1.9 |
| 30 | (IBN)₃NO | 140 | S-100 | 70.0 | 3000 | 2.1 |
| 31 | (MBN)₃NO | 140 | S-100 | 70.9 | 2700 | 2.1 |
| K | Trigonox21 | 140 | S-100 | 70.8 | 3200 | 2.1 |
| nBuAc = n-butylacetate | | | | | | |
| S-100 = Solvesso® 100 | | | | | | |

The initers according to the invention allow the production of a high solids resin at various temperatures in good yields with a molecular weight that is lower than observed in conventional processes.

### Example 32

In an open flask, 3.3 mol/l styrene (18 g) in ODCB was polymerized at 140°C for 6 hours, using 9 g of the product obtained in Example 3 as the initer and 0.027 mol/l CSA. The following results were obtained:

| Time | Conversion | Mn | D |
|---|---|---|---|
| (min) | (%w/w) | (Dalton) | |
| 0 | 0 | - | |
| 45 | 14.8 | 8620 | 2.0 |
| 90 | 30.2 | 10100 | 2.2 |
| 135 | 41.7 | 11050 | 2.2 |
| 180 | 51.3 | 12070 | 2.3 |
| 225 | 58.9 | 12830 | 2.2 |
| 270 | 65.4 | 13170 | 2.3 |
| 315 | 70.7 | 13530 | 2.3 |
| 360 | 74.6 | 13730 | 2.3 |

Characterization of the polymer showed the expected product to be formed.

### Example 33

The previous example was repeated, except that the initer of Example 3 was replaced by the initer obtained in Example 13, with the following results.

| Time | Conversion | Mn | D |
|---|---|---|---|
| (min) | (%w/w) | (Dalton) | |
| 0 | 1.2 | 3170 | 1.8 |
| 45 | 6.7 | 3590 | 2.1 |
| 90 | 12.7 | 4200 | 2.4 |
| 135 | 16.7 | 4650 | 2.5 |
| 180 | 21.3 | 5060 | 2.5 |
| 225 | 25.0 | 5320 | 2.6 |
| 270 | 27.4 | 5650 | 2.5 |
| 315 | 28.9 | 5930 | 2.5 |
| 360 | 31.2 | 6070 | 2.5 |

Again it was shown with 1H-NMR that the expected copolymer was formed.

## Claims

1. A pseudo-living polymerization process in which at least one NO-compound with at least one moiety according to formula (I) or (II), 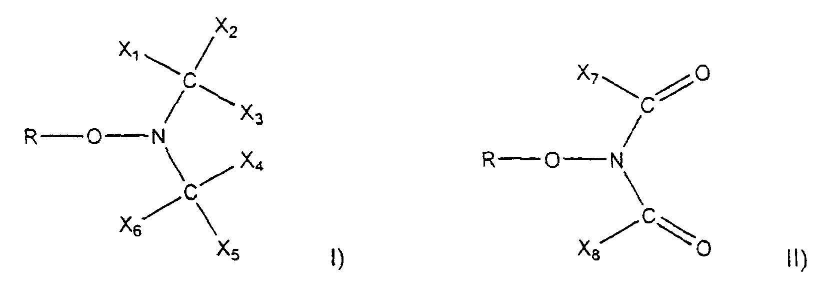 wherein:
• R represents a group which has at least one carbon atom and is such that the free radical R• is capable of initiating the free radical polymerization of unsaturated monomers;
• at most five of the groups represented by X₁-X₆ are the same or different straight-chain or branched, substituted or unsubstituted (cyclo) alkyl groups, while two or more of the groups X₁-X₆ may be linked to form cyclic structures,
• the complementary groups X₁ through X₆ are functional groups selected from substituted or unsubstituted phenyl, cyano, ether, hydroxy, nitro, dialkoxyphosphonyl, and carbonyl containing groups, or, alternatively, -CX₁X₂X₃ and/or -CX₄X₅X₆ represent a phenyl group,
• X₇ and X₈ are independently selected from alkyl, aryl, alkaryl, and aralkyl, while X₇ is optionally linked with X₈ to form bridged structures, and
is combined with an ethylenically unsaturated monomer composition to be polymerized.

2. A process according to claim1 wherein the complementary functional groups X₁-X₆ are phenyl or cyano.

3. A process according to claim 1 or 2 wherein two or more ethylenically unsaturated monomers are polymerized either sequentially or simultaneously.

4. A process according to any one of the preceding claims, **characterized in that** the ethylenically unsaturated monomers are selected from styrene, divinylbenzene, α-methylstyrene, chloromethylstyrene, acrylic acid, esters of acrylic acid, methacrylic acid, esters of methacrylic acid, maleic acid, maleic anhydride, esters of maleic acid, fumaric acid, esters of fumaric acid, acrylonitrile, vinylpyridine, isoprene, butadiene, trienes, styrene phosphonic acid, vinyl phosphonic acid, styrene phosphonic esters, vinyl phosphonic acid esters, maleimides, citraconimides, itaconimides, vinylacetate, their derivatives, and substituted analogues.

5. A process according to any any one of claims 1-4, **characterized in that** the reactants do not comprise free radical generating species other than one or more NO-compounds of formula I or II.

6. A process according to any one of claims 1-5, **characterized in that** the molecular weight (distribution) of the resulting polymer is controlled by the concentration of monomer and NO-compound.

7. A process according to any one of claims 1-6 wherein an additional stable free radical is used.

8. A process according to claim 7 wherein the stable free radical is similar to the nitroxide radical that is formed when one or more of the applied NO-compounds is thermally decomposed.

9. A (block) (co)polymer obtainable by any one of the processes of claims 1-8.

10. An ND-compound containing at least one moiety of the formula (I), 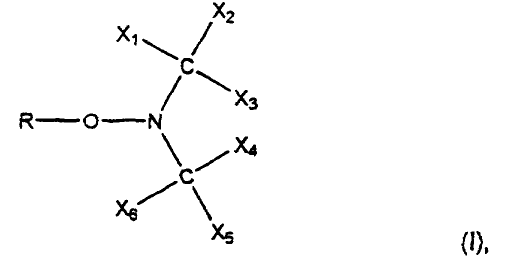 wherein:
• R represents a group which has at least one carbon atom and is such that the free radical R• is capable of initiating the free radical polymerization of unsaturated monomers;
• at most five of the groups represented by X₁ through X₆ are the same or different straight-chain or branched substituted or unsubstituted (cyclo) alkyl groups, while two or more of the groups may be linked to form cyclic structures,
• the complementary groups X₁ through X₆ are functional groups selected from substituted or unsubstituted phenyl, cyano, ether, hydroxy, nitro, dialkoxyphosphonyl, and carbonyl containing groups, such as ester, carboxyalkyl, aldehyde, anhydride, and ketoalk(ar)yl groups, and
• R is not polymeric when -N(CX₁X₂X₃) CX₄X₅X₆ is 2,5-dimethyl-2,5-diphenylpyrrolidin-1-,
with the proviso that the NO-compound is not tris-(2-cyano-2-propyl)-hydroxylamine, tris-(2-carboxyethyl-2-propyl) hydroxylamine, tris-(1-cyanocyclohexyl) hydroxylamine, and/or tris-(2-carboxymethyl-2-propyl) hydroxylamine.

11. An NO-compound according to claim 10, **characterized in that** the complementary groups X are selected from substituted or unsubstituted phenyl, cyano, ether, and carbonyl containing groups, such as ester, anhydride, and ketoalk(ar)yl groups.

12. A recycling process in which a polymer is converted into low-molecular weight fragments, **characterized in that** the polymer is obtained from a pseudo-living polymerization process according to any one of claims 1-8.

13. A recycling process according to claim 12, **characterized in that** the processing temperature is below 300°C.

## Patentansprüche

1. Verfahren der pseudo-lebenden Polymerisation, in dem wenigstens eine NO-Verbindung, die wenigstens einen Rest gemäß den Formeln (I) oder (II): 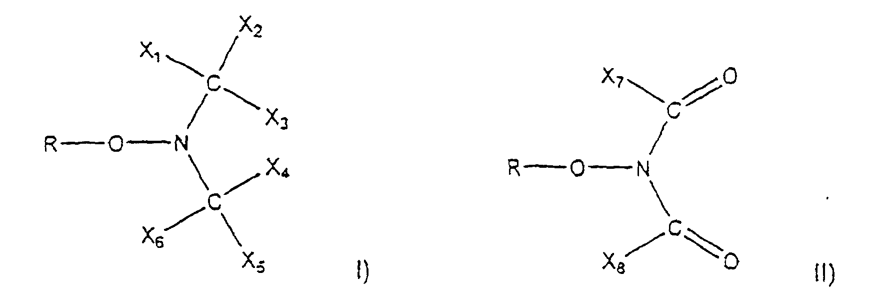 aufweist, worin
• R eine Gruppe darstellt, die wenigstens ein Kohlenstoffatom aufweist und derartig ist, dass das Radikal R· zur Initiierung der radikalischen Polymerisation ungesättigter Monomere befähigt ist;
• höchstens fünf der durch X₁-X₆ dargestellten Gruppen einander gleich sind oder unterschiedliche geradkettige oder verzweigtkettige, substituierte oder unsubstituierte (Cyclo)-alkylgruppen sind, während zwei oder mehrere der Gruppen X₁-X₆ verbunden sein können, um cyclische Strukturen zu bilden;
• die komplementären Gruppen X₁ bis X₆ funktionelle Gruppen sind, die aus substituierten oder unsubstituierten Phenyl-, Cyano-, Ether-, Hydroxy-, Nitro-, Dialkoxyphosphonyl- und Carbonyl-enthaltenden Gruppen ausgewählt sind, oder alternativ dazu -CX₁X₂X₃ und/oder -CX₄X₅X₆ eine Phenylgruppe darstellen;
• X₇ und X₈ unabhängig voneinander aus Alkyl, Aryl, Alkaryl und Aralkyl ausgewählt sind, wobei X₇ gegebenenfalls mit X₈ verbunden ist, um überbrückte Strukturen zu bilden;
mit einer zu polymerisierenden, ethylenisch ungesättigten Monomerzusammensetzung kombiniert wird.

2. Verfahren gemäß Anspruch 1, worin die komplementären, funktionellen Gruppen X₁-X₆ Phenyl oder Cyano sind.

3. Verfahren gemäß den Ansprüchen 1 oder 2, worin zwei oder mehrere ethylenisch ungesättigte Monomere entweder nacheinander oder gleichzeitig polymerisiert werden.

4. Verfahren gemäß irgendeinem der vorhergehenden Ansprüche, **dadurch gekennzeichnet, dass** die ethylenisch ungesättigten Monomere aus Styrol, Divinylbenzol, α-Methylstyrol, Chloromethylstyrol, Acrylsäure, Estern der Acrylsäure, Methacrylsäure, Estern der Methacrylsäure, Maleinsäure, Maleinsäureanhydrid, Estern der Maleinsäure, Fumarsäure, Estern der Fumarsäure, Acrylnitril, Vinylpyridin, Isopren, Butadien, Trienen, Styrolphosphonsäure, Vinylphosphonsäure, Styrolphosphonsäureestern, Vinylphosphonsäureestern, Maleimiden, Citraconimiden, Itaconimiden, Vinylacetat, deren Derivaten und substituierten Analogen ausgewählt sind.

5. Verfahren gemäß irgendeinem der Ansprüche 1 bis 4, **dadurch gekennzeichnet, dass** die Reaktanten nicht solche Radikal-erzeugende Spezies umfassen, die von einer oder mehreren NO-Verbindungen der Formeln I oder II verschieden sind.

6. Verfahren gemäß irgendeinem der Ansprüche 1-5, **dadurch gekennzeichnet, dass** die Molmasse (Molmassenverteilung) des sich ergebenden Polymers durch die Konzentration von Monomer und NO-Verbindung gesteuert wird.

7. Verfahren gemäß irgendeinem der Ansprüche 1-6, worin ein zusätzliches stabiles Radikal verwendet wird.

8. Verfahren gemäß Anspruch 7, worin das stabile Radikal dem Stickstoffoxid ("nitroxide")-Radikal ähnlich ist, das gebildet wird, wenn eine oder mehrere der verwendeten NO-Verbindungen thermisch zersetzt werden.

9. (Block)(co)polymer, das durch irgendeines der Verfahren der Ansprüche 1 bis 8 erhältlich ist.

10. NO-Verbindung, die wenigstens einen Rest gemäß der Formel (I) : 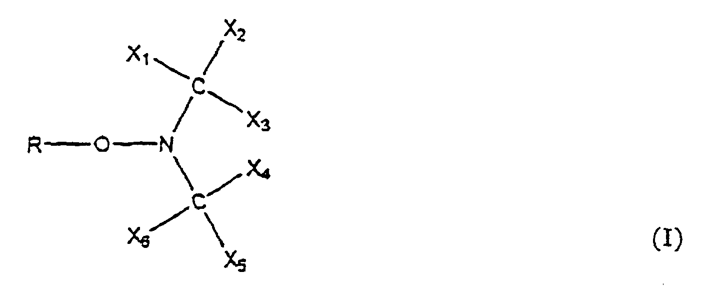 enthält, worin
• R eine Gruppe darstellt, die wenigstens ein Kohlenstoffatom aufweist und derartig ist, dass das Radikal R· zur Initiierung der radikalischen Polymerisation ungesättigter Monomere befähigt ist;
• höchstens fünf der durch X₁-X₆ dargestellten Gruppen einander gleich sind oder unterschiedliche geradkettige oder verzweigtkettige, substituierte oder unsubstituierte (Cyclo)-alkylgruppen sind, während zwei oder mehrere der Gruppen verbunden sein können, um cyclische Strukturen zu bilden;
• die komplementären Gruppen X₁ bis X₆ funktionelle Gruppen sind, die aus substituierten oder unsubstituierten Phenyl-, Cyano-, Ether-, Hydroxy-, Nitro-, Dialkoxyphosphonyl- und Carbonyl-enthaltenden Gruppen, wie Ester-, Carboxyalkyl-, Aldehyd-, Anhydrid- und Ketoalk(ar)yl-Gruppen ausgewählt sind, und
• R nicht polymer ist, wenn -N(CX₁X₂X₃)CX₄X₅X₆ 2,5-Dimethyl-2,5-diphenylpyrrolidin-1- ist;
mit der Maßgabe, dass die NO-Verbindung nicht tris(2-Cyano-2-propyl)hydroxylamin, tris(2-Carboxyethyl-2-propyl)hydroxylamin, tris(1-Cyanocyclohexyl)hydroxylamin und/oder tris(2-Carboxymethyl-2-propyl)hydroxylamin ist.

11. NO-Verbindung gemäß Anspruch 10, **dadurch gekennzeichnet, dass** die komplementären Gruppen X aus substituierten oder unsubstituierten Phenyl-, Cyano-, Ether- und Carbonyl-enthaltenden Gruppen, wie Ester-, Anhydrid- und Ketoalk(ar)yl-Gruppen ausgewählt sind.

12. Recycling-Verfahren, in dem ein Polymer in Fragmente niedriger Molmasse überführt wird, **dadurch gekennzeichnet, dass** das Polymer aus einem Verfahren der pseudo-lebenden Polymerisation gemäß irgendeinem der Ansprüche 1 bis 8 erhalten wird.

13. Recycling-Verfahren gemäß Anspruch 12, **dadurch gekennzeichnet, dass** die Verarbeitungstemperatur unterhalb von 300 °C liegt.

## Revendications

1. Procédé de polymérisation pseudo-vivante dans lequel au moins un composé de NO ayant au moins un motif selon la formule (I) ou (II), 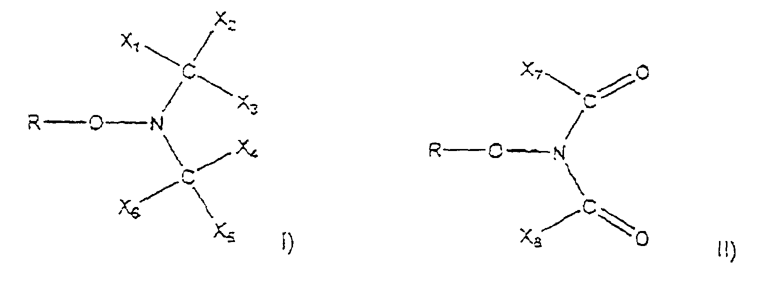 dans laquelle :
• R représente un groupement ayant au moins un atome de carbone et est tel que le radical libre R· est capable d'initier la polymérisation radicalaire de monomères insaturés ;
• cinq au plus des groupements représentés par X₁-X₆ sont des groupements (cyclo)alkyles, substitués ou non substitués, à chaîne linéaire ou branchée, identiques ou différents, tandis que deux ou plus des groupements X₁-X₆ peuvent être liés pour former des structures cycliques ;
• les groupements complémentaires X₁ à X₆ représentent des groupements fonctionnels choisis parmi des groupements phényle substitué ou non substitué, cyano, éther, hydroxy, nitro et dialkoxyphosphonyle et des groupements contenant un carbonyle ou, alternativement, -CX₁X₂X₃ et/ou -CX₄X₅X₆ représentent un groupement phényle ;
• X₇ et X₈ sont choisis indépendamment parmi un alkyle, un aryle, un alkaryle et un aralkyle, tandis que X₇ est éventuellement lié avec X₈ pour former des structures pontées, et
est associée à une composition de monomères éthylèniquement insaturés devant être polymérisée.

2. Procédé selon la revendication 1, dans lequel les groupements fonctionnels complémentaires X₁-X₆ représentent le phényle ou le cyano.

3. Procédé selon la revendication 1 ou 2, dans lequel deux ou plusieurs monomères éthylèniquement insaturés sont polymérisés, soit séquentiellement soit simultanément.

4. Procédé selon l'une quelconque des revendications précédentes, **caractérisé en ce que** les monomères éthylèniquement insaturés sont choisis parmi le styrène, le divinylbenzène, l'α-méthylstyrène, le chlorométhylstyrène, l'acide acrylique, les esters de l'acide acrylique, l'acide méthacrylique, les esters de l'acide méthacrylique, l'acide maléique, l'anhydride maléique, les esters de l'acide maléique, l'acide fumarique, les esters de l'acide fumarique, l'acrylonitrile, la vinylpyridine, l'isoprène, le butadiène, les triènes, l'acide styrènephosphonique, l'acide vinylphosphonique, les esters styrènephosphoniques, les esters de l'acide vinylphosphonique, les maléimides, les citraconimides, les itaconimides, l'acétate de vinyle, leurs dérivés et analogues substitués.

5. Procédé selon l'une quelconque des revendications 1 à 4, **caractérisé en ce que** les agents réactifs ne comportent pas les espèces génératrices de radicaux libres autres qu'un ou plusieurs des composés de NO de formule I ou II.

6. Procédé selon l'une quelconque des revendications 1 à 5, **caractérisé en ce que** le poids moléculaire (répartition)du polymère résultant est contrôlé par la concentration en monomères et composé de NO.

7. Procédé selon l'une quelconque des revendications 1 à 6, dans lequel un radical libre stable supplémentaire est utilisé.

8. Procédé selon la revendication 7, dans lequel le radical libre stable est semblable au radical nitroxyde qui est formé lorsqu'un ou plusieurs des composés de NO appliqués sont décomposés thermiquement.

9. (Co)polymère (en bloc)pouvant être obtenu par l'un quelconque des procédés des revendications 1 à 8.

10. Composé de NO contenant au moins un motif de formule (I), 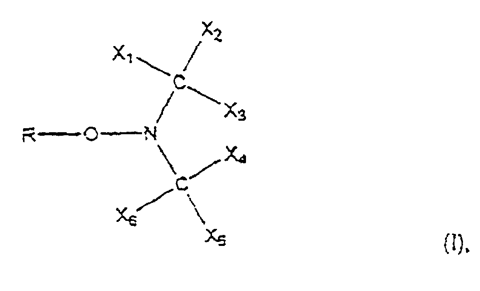 dans laquelle :
• R représente un groupement ayant au moins un atome de carbone et est tel que le radical libre R· est capable d'initier la polymérisation radicalaire de monomères insaturés ;
• cinq au plus des groupements représentés par X₁ à X₆ sont des groupements (cyclo)alkyles, substitués ou non substitués, à chaîne linéaire ou branchée, identiques ou différents, tandis que deux ou plus des groupements peuvent être liés pour former des structures cycliques ;
• les groupements complémentaires X₁ à X₆ représentent des groupements fonctionnels choisis parmi des groupements phényle substitué ou non substitué, cyano, éther, hydroxy, nitro et dialkoxyphosphonyle et des groupements contenant un carbonyle, tels que des groupements ester, carboxyalkyle, aldéhyde, anhydride et cétoalk(ar)yle, et
• R n'est pas polymérique lorsque -N(CX₁X₂X₃)CX₄X₅X₆ représente la 2,5-diméthyl-2,5-diphénylpyrrolidin-1-,
à condition que le composé de NO n'est pas la tris-(2-cyano-2-propyl)hydroxylamine, la tris-(2-carboxye-thyl-2-propyl)hydroxylamine, la tris-(1-cyanocyclohexyl)hydroxylamine et/ou la tris-(2-carboxyméthyl-2-propyl)hydroxylamine.

11. Composé de NO selon la revendication 10, **caractérisé en ce que** les groupements complémentaires X sont choisis parmi des groupements phényle substitué ou non substitué, cyano et éther et des groupements contenant un carbonyle, tels que des groupements ester, anhydride et cétoalk(ar)yle.

12. Procédé de recyclage dans lequel un polymère est converti en fragments de bas poids moléculaire, **caractérisé en ce que** le polymère est obtenu par un procédé de polymérisation pseudo-vivante selon l'une quelconque des revendications 1 à 8.

13. Procédé de recyclage selon la revendication 12, **caractérisé en ce que** la température de transformation est inférieure à 300°C.
